(19) **Europäisches Patentamt European Patent Office Office européen des brevets**

(11) **EP 1 267 830 B1**

(12) **EUROPEAN PATENT SPECIFICATION**

(45) Date of publication and mention
of the grant of the patent:
**27.05.2009 Bulletin 2009/22**

(51) Int Cl.:
**A61K 9/107** *(2006.01)*     **A61Q 19/00** *(2006.01)*

(21) Application number: **01918930.7**

(86) International application number:
**PCT/US2001/009272**

(22) Date of filing: **23.03.2001**

(87) International publication number:
**WO 2001/070197 (27.09.2001 Gazette 2001/39)**

(54) **METHOD FOR PREPARING HIGH PRESSURE/HIGH SHEAR DISPERSIONS CONTAINING PHYSIOLOGICALLY ACTIVE INGREDIENTS**

VERFAHREN ZUR HERSTELLUNG VON PHYSIOLOGISCH AKTIVEN SUBSTANZEN ENTHALTENDEN DISPERSIONEN UNTER ANWENDUNG VON HOCHDRUCK UND HOCHSCHERKRAFT

PROCEDE A CISAILLEMENT ELEVE/PRESSION ELEVEE POUR LA PREPARATION DE DISPERSIONS CONTENANT DES INGREDIENTS ACTIFS AU PLAN PHYSIOLOGIQUE

(84) Designated Contracting States:
**AT BE CH CY DE DK ES FI FR GB GR IE IT LI LU MC NL PT SE TR**

(30) Priority: **23.03.2000 US 191508 P**

(43) Date of publication of application:
**02.01.2003 Bulletin 2003/01**

(73) Proprietor: **BASF Catalysts LLC
Iselin, NJ 08830-0770 (US)**

(72) Inventor: **WILMOTT, James, M.
Shoreham, NY 11786 (US)**

(74) Representative: **Olsen, Lars Pallisgaard et al
Zacco Denmark A/S
Hans Bekkevolds Allé 7
2900 Hellerup (DK)**

(56) References cited:
**WO-A-98/14174          WO-A-99/26599
US-A- 6 004 566**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates to methods for preparing dispersions containing physiologically active ingredients.

### BACKGROUND OF THE INVENTION

Most topical preparations currently produced contain a wide variety of physiologically active ingredients and/or aesthetic modifiers. Physiologically active ingredients are compounds which cause a physical change to the body following their application. Examples of such ingredients include alpha hydroxy acids, antioxidants and vitamins. Aesthetic modifiers provide the composition with a defined physical characteristic such as, for example, the degree of moisturization, oil content, and physical form of the composition.

Some examples of aesthetic modifiers include silicone fluids and derivatives, waxes, botanical (vegetable) oils, hydrocarbon-based oils, esters and fragrances. The performance of these ingredients is dependent upon the vehicle used to deliver them. These vehicles range from simple solvents, such as water or ethanol, to complex emulsions.

Unfortunately not all active ingredients are completely soluble or compatible with all vehicles. For example, oil soluble active ingredients are typically not compatible with water or water-based gel vehicles. As a result, many products exhibit poor delivery of the active ingredients, have poor tactile properties, or are thermodynamically unstable and result in a commercially unacceptable shelf life. Non-water based solvents can also be used as a vehicle for hydrophilic physiologically active materials or aesthetic modifiers. However, these preparations are typically not cosmetically elegant Further, these non-water based solvents can cause unwanted side effects such as irritation or damage to the epithelial surface to which they are applied.

High pressure high shear dispersions are finding increasing application in cosmetic, personal care, over-the-counter (OTC), Rx, nutritional and food products. These systems can be mixed into a compatible base to create products with superior performance and aesthetic properties.

Generally, a dispersion is formed by dispersing a hydrophobic phase into a polar, hydrophilic phase, which is principally water. However, if the material to be dispersed has too much polarity, or if it is solid in its native state it will not disperse readily and therefore is excluded from use in developing new treatment products. This situation is unfortunate and does not allow for the preparation of physiologically efficacious products using materials that can treat a particular disorder but which are not water dispersible in their own right.

Many physiologically active agents are unable to disperse directly into an aqueous phase. These materials usually are simply dissolved into solvents before they are applied to the surface to be treated. These solvent systems are usually hydrocarbon based materials of varying polarity. The solvent is selected based on its ability to dissolve the physiologically active material of choice to treat a particular topical disorder. These solvent systems often are irritating, can damage the surface to which they are applied and are very unaesthetic. Further, if the physiologically active material is unstable to conventional processing methods, an alternative method of introducing the material into a product is necessary to maintain its beneficial properties.

To overcome the negative properties usually associated with the use of simple aqueous or non-water based solvents, a formulator typically uses stable dispersions to deliver the physiologically active ingredient or aesthetic modifier to the epithelial surface to be treated. These dispersions form either spherical micelles of one or more hydrophobic liquid materials in water or spherical droplets of water in a hydrophobic fluid. Such dispersions are typically prepared by creating the oil phase and water phase then mixing the two phases together.

Specifically, the hydrophobic physiologically active ingredients or aesthetic modifiers are dissolved in a suitable oil phase and the hydrophilic physiologically active ingredients or aesthetic modifiers are dissolved in water, and then the two phases are combined with one or more emulsifying agents which are incorporated into either or both the water and oil phases. These emulsifiers are surface active agents (surfactants) whose role is to reduce the surface tension between the oil and water phases thereby making the combination of the two phases more stable. Such "emulsions" are generally prepared by heating the oil and water phases to elevated temperatures exceeding 70-75°C before combining, then slowly cooling the combined phases to ensure the development of the suitable crystalline and liquid crystalline structures which gives the emulsion its characteristic properties. These emulsions usually have a homogeneous opaque white appearance and a smooth or pleasant feeling upon application to the skin or other epithelial surface. However, the use of typical emulsion products to deliver physiological or aesthetic benefits has many limitations.

The presence of significant amounts of surfactant can strip material from the lipid barrier of the skin or the lipid bilayer of epithelial cell membranes leaving the tissue vulnerable. Thus, the surfactants themselves can evoke an irritation or the damaged barrier will permit the passage of other materials that can cause irritation or increase skin sensitivity and allergic reactions, The literature is replete with clinical evidence of the damaging consequences that can occur with the use or overuse of surfactants. For example, Effendy I, Maibach H I, "Surfactants and experimental irritant contact dermatitis", Contact Dermatitis 1995 October; 33(4); 217-25 indicates that "[m]any surfactants elicit irritant reactions when applied to the skin, partially due to their relative ability to solubilize lipid membranes."; Barany E, Lindberg M, Loden M, "Biophysical characterization of skin damage and recovery after exposure to different surfactants", Contact Dermatitis 1999 February;40(2):98-103, states that "[t]he majority of adverse skin reactions to personal-care products are presumed to be caused by irritant substances, like surfactants."

Moreover, there are limitations to conventional topical formulations. For example, many materials with interesting aesthetic properties are not easily produced in an emulsion such as, for example, fluorinated compounds. Additionally, each time the oil or water phase is changed, the emulsifiers would need to be rebalanced. The incorporation of additional materials using conventional techniques can affect surface tension adversely, leading to the final product becoming unstable.

Many topical products formulated contain active ingredients and/or certain aesthetic modifiers which readily become destabilized in emulsions, causing them to degenerate or deteriorate. For example, prolonged heating of the water and oil phases can thermodynamically modify the active molecule or can kinetically accelerate the reaction of the active with another agent in the emulsion or with air if the material is oxygen sensitive. Moreover, lowering the surface tension of a topical composition generally increases the surface exposure of the active or sensitive aesthetic modifier to oxygen and other destabilizing materials. For example, when retinol is the active ingredient, the instability of the composition leads to lower efficacy. The instability of an unsaturated fatty acid as an aesthetic modifier leads to color changes and malodors in the composition. Since the time between manufacturing and sale of a cosmetic product is typically several weeks, the product is often no longer "fresh" or effective since the active ingredient has degenerated or deteriorated. To offset instability problems, many other materials such as chelating agents, antioxidants and masking agents are usually included in the formulation.

Typical emulsions are time consuming to prepare, require heating, are produced in multiple phases, are slow cooling, and often require high shear conditions to get the particle size small enough for maximum stability. Larger batches may take from 8 to 24 hours to process and can take several days to set up. It is also very difficult to get excellent reproducibility of an emulsion. If any factors such as the heating, cooling or mixing rates are not carefully duplicated, the preparation may have different properties than the preceding batches of the same product. Often the difference of a single parameter is significant enough to cause the product to be outside the established optimum specifications. These batches then have to be either discarded or re-worked.

The lack of reproducibility is especially problematic when the product contains a drug or other physiologically active ingredient. Lack of reproducibility can effect product performance and end user satisfaction. The lack of reproducibility will result in products from different batches having different aesthetic properties which the end user will perceive as a lack of quality and will ultimately lead to consumer dissatisfaction or reduced compliance.

Standard emulsion preparations also have a high cost to manufacture. This is due to a variety of factors including the energy to heat the batch, the specialized equipment required to process the emulsion such as specialized pumps and cooling/heating equipment and the length of process ties up equipment and personnel, resulting in increased overhead and lost opportunity time.

WO 98/14174 A discloses a method of forming a dispersion of a water-insoluble pharmacologically active agent which dispersion contains substantially no surfactants, comprising the steps of mixing a solution of the active agent with the aqueous medium containing biocompatible polymer and subjecting the composition to homogenisation under high pressure and high shear forces in the presence of an aqueous system. The particle size in the resulting dispersion is not greater than 1 micron.

US 6,004,566 discloses a similar method of forming an emulsion of e.g. a lipophilic compound in an aqueous phase by the use of high shear high pressure mixing, but the method of US 6,004,566 includes the addition of a surfactant. There is no indication in US 6,004,566 of which particle size is obtained.

Applicants have now discovered a method of forming stable dispersions of physiologically active agents, comprising dispersing a hydrophobic phase into a polar hydrophilic phase which is principally water. The hydrophobic phase, which is nonpolar or low polar, is combined with the aqueous phase using high pressure high shear conditions to create a stable dispersion.

OBJECTS OF THE INVENTION

Applicants have discovered a method of making more efficacious formulations than those obtained by prior art methods.

Applicants have discovered a method of forming compositions having greater ingredient stability than prior art compositions.

Applicants have discovered a method of forming compositions which are more cosmetically elegant and less irritating than prior art formulations.

The method of the invention also provides greater flexibility in obtaining formulations, and substituting ingredients, and allows the formulator to disregard HLB rebalancing which is often a problem with the changes to formulations in the prior art. The method of the invention permits easier scale up to manufacturing.

The method of the invention involves reduced manufacturing costs by reducing processing time and energy costs and lower capital investment in equipment.

The method of the invention results in much more consistent reproducibility than prior art methods, causing less wasted batches and work-off.

## SUMMARY OF THE INVENTION

In one embodiment, the invention is directed to a method of forming a dispersion of a nonpolar or slightly polar physiologically active agent in an aqueous composition and which is free of emulsifying surfactants. The method of the invention comprises the steps of mixing said nonpolar or slightly polar physiologically active agent with a solvent or cosolvent, combining said mixture with water and subjecting said combination to high pressure/high shear mixing to form a stable dispersion, with a particle size of from about 50 to about 1000 nm. In other embodiments, the dispersion may have a particle size of from about 50 to about 500 nm.

The invention is also directed to a method of forming a dispersion of a nonpolar or slightly polar physiologically active agent in an aqueous composition and which is free of emulsifying surfactants, said method consisting of the steps of

a) mixing said nonpolar or slightly polar physiological active agent with a solvent or cosolvent;
b) combining said mixture with water;
c) subjecting said combination to high pressure and/or high shear mixing to form a stable dispersion with a particle size of from about 50 to about 1000 nm.

The invention is also related to a method of forming a dispersion of a nonpolar or slightly polar physiologically active agent in an aqueous composition and which is free of emulsifying surfactants, said method comprising the steps of

a) mixing said nonpolar or slightly polar physiological active agent with a solvent or cosolvent selected from the group consisting of soybean oil, octylmethoxycinnamate, octyl salicylate and cyclomethicone;
b) combining said mixture with water;
c) subjecting said combination to high pressure and/or high shear mixing to form a stable dispersion with a particle size of from about 50 to about 1000 nm.

## DETAILED DESCRIPTION OF THE INVENTION

A hydrophobic active ingredient or hydrophobic adjuvant of the present invention is an active ingredient or adjuvant which has a non polar property which makes it essentially insoluble in water or water and polar solvent solutions. Hydrophobic active ingredients of the present invention include, but are not limited to, partially and fully hydrophobic active ingredients. For example, hydrophobic active ingredients encompassed by the present invention include compounds and complexes which contain a hydrophobic moiety.

The topical preparation of the present invention may also include non-hydrophobic active ingredients and non-hydrophobic adjuvants.

Suitable active agents include, but are not limited to, anti-acne agents, antimicrobial agents, antiinflammatory agents, analgesics, antietythemal agents, antipruritic agents, antiedemal agents, antipsoriatic agents, antifungal agents, skin protectants, sunscreen agents, vitamins, antioxidants, scavengers, antiirritants, antibacterial agents, antiviral agents, antiaging agents, protoprotection agents, hair growth enhancers, hair growth inhibitors, hair removal agents, antidandruff agents, anti-seborrheic agents, exfoliating agents, wound heating agents, anti-ectoparacitic agents, sebum modulators, immunomodulators, hormones, botanicals, moisturizers, astringents, sensates, antibiotics, anesthetics, steroids, tissue heating substances, tissue regenerating substances, amino acids, peptides, minerals, ceramides, biohyaluronic acids, and any combination of any of the foregoing.

Preferred anti-acne agents include, but are not limited to, salicylic acid, retinoic acid, alpha hydroxy acid, benzyl peroxide, sodium sulfacetamide, clindamycin, and any combination of any of the foregoing. Preferred combinations of anti-acne agents to be incorporated in the composition include salicylic acid, retinoic acid, and hydrocortisone; sodium sulfacetamide and clindamycin; salicylic acid and clindamycin; salicylic acid, alpha hydroxy acid, and tetrahydrozoline.

Suitable antimicrobial agents include, but are not limited to, Benzalkonium chloride, Benzethonium chloride, Chlorhexidine gluconate, Chloroxylenol, Clindamycin Cloflucarban, erythromycin, Fluorosalan, Hexachlorophene, Hexylresorcinol, Iodine complex, Iodine tincture, Para-chloromercuriphenol, Phenylmercuric nitrate, Thimerosal, Vitromersol, Zyloxin, Triclocarban, Triclosan, Methyl-benzethonium chloride, Nonyl phenoxypoly (ethyleneoxy) ethanol-iodine, Para-chloro-meta-xylenol, Providone-iodine complex, Poloxamer-iodine complex, Triclorcarban, Undecoylium chloride-iodine complex, and any combination of any of the foregoing.

Suitable antiinflammatory agents include, but are not limited to, Alidoxa, Allantoin, Aloe Vera, Aluminum acetate, Aluminum hydroxide, Bismuth subnitrate, Boric acid, Calamine, Casein, Cellulose, microporous, Cholecatciferol, Cocoa butter, Cod liver oil, Colloidal oatmeal, Cystein hydrochloride, Dexpanthenol, Dimethicone, Glycerin, Kaolin, Lanolin, Live yeast cell derivative, Mineral oil, Peruvian balsam, Petrolatum, Protein hydrolysate, Racemethionine, Shark liver oil, Sodium bicarbonate, Sulfur, Talc, Tannic acid, Topical starch, Vitamin A, Vitamin E, White petrolatum, Zinc acetate, Zinc carbonate, Zinc oxide, Hydrocortisone, Betamethasone, Ibuprofen, Indomethicin, Acetyl salicylic acid, Tacrolimus, Flucoinolone acetonide, Sodium sulfacetamide, and any combination of any of the foregoing.

The compositions of the invention may include a wide range of active agents having various anti-irritation and anti-inflammatory activities. Suitable physiologically active agents which are too polar to be effectively dispersed in an aqueous (or hydrophilic) phase are the following:

Sansurf® Shea butter, Sansur® DMG and Dermaguard, which have desirable barrier properties;
Silox chamomile, sea salt, A/I liposomes, sea parsley, Sansurf® Shea Butter, InCyte® Lemon Peel, Melarnest™ L, and Bisabolol SS, which act to block signal development;
ExCyte® Hops and Melarrest™ L, which block recruitment;
ExCyte® Hops, sea parsley and Heather ExCyte®, which act as MMP suppression and block neutralization agents;
MPC, Sansurf® EFA, Sansurf® oils, ceramides, sphingolipids and liposomes, which act as barrier repairs;
Hyaluronic acid quaternatery compounds, MCP, HA-SOL, AMC, Seamollient®, Botanigels, Categel, moisturizations liposomes, and humectant liposomes, which act as humectants;
MPC, Moistureguard, vegepure, Sansurf® oils and polyfix, which act as occlusive barrier agents;
Solarease™ OMC/1789, Solarcat™ OMC/1789 and TioSperse™ Ultra, which act as UV abosorbers;
A/O complex, silox GT, lemon balm, Excite® green tea, PhoCyte® lemon peel, InCyte® apple and InCyte® kola, which act as anti-oxidants; and
beta glucan, which enhances the immune system (acts as an immune stimulator and enhancement).

Suitable analgesics include, but are not limited to, diphenhydramine, tripelennamine, benzocaine, dibucaine, lidocaine, tetracaine, camphor, menthol, phenol, resorcinol, matacresol, juniper tar, methylsalicylate, turpentine oil, capsicum, methyl nicotinate, beta-glucan, and any combination of any of the foregoing.

Suitable antierythemal agents include, but is not limited to, tetrahydrozoline and hydrocortisone.

Suitable antipruritic agents include, but are not limited to, benadryl, pramoxine, antihistamines, and any combination of any of the foregoing.

Suitable antiedemal agents, include, but are not limited to, pregnenalone acetate, tanin glyrosides, and any combination of any of the foregoing.

Suitable antipsoriatic agents include, but are not limited to, caleipotriene, coal tar, anthralin, vitamin A, and any combination of any of the foregoing. Preferred combinations of antipsoriatic agents include, but are not limited to, hydrocortisone, retinoic acid, and alpha hydroxy acid; dovonex, salicylic acid, and a sunscreen agent; indomethicin, salicylic acid, and urea; anthralin and salicylic acid; and anthralin and indomethicin. Other suitable antipsoriatic agents include, but are not limited to, caleipotriene, coal tar, anthralin, vitamin A, and any combination of any of the foregoing.

Suitable antifungal agents include, but are not limited to, clioquinol, haloprogin, miconazole nitrate, clotrimazole, metronidazole, tolnaftate, undecylenic acid, iodoquinol, and any combination of any of the foregoing.

Suitable skin protectants include, but are not limited to, cocoa butter, dimethicone, petrolatum, white petrolatum, glycerin, shark liver oil, allantoin, and any combination of any of the foregoing.

Suitable sunscreen agents include, but are not limited to, octyl methoxycinnamate, avobenzone, beazophenone-3, octacrylene, titanium dioxide, zinc oxide, and any combination of any of the foregoing.

A preferred sunscreen agent is a mixture of octylmethoxycinnamate, butyl methoxydibenzoylmethane, cyclomethicone, one or more phospholipids and water, and is available as Solarease™ from Collaborative Laboratories, Inc. of Stony Brook, New York.

Suitable antioxidants include, but are not limited to, scavengers for lipid free radicals and peroxyl radicals, quenching agents, and any combination of any of the foregoing.

Suitable antioxidants include, but are not limited to, tocopherol, BHT, beta carotene, vitamin A, ascorbic acid, ubiquinol, ferulic acid, azelaic acid, thymol, catechin, sinapic acid, EDTA, lactoferrin, rosmariquinone, hydroxytyrosole, sesamol, 2-thioxanthine, nausin, malvin, carvacone, chalcones, glutathione isopropyl ester, xanthine, melanin, guanisone, lophorphyrins, 8-hydroxyxanthine, 2-thioxanthione, vitamin $B_{12}$, plant alkaloids, catalase, quercetin, tyrosine, SOD, cysteine, methionine, genistein, NDGA, procyanidin, hamamelitannin, ubiquinone, trolox, licorice extract, propyl gallate, sinapic acid, and any combination of any of the foregoing.

Suitable vitamins include, but are not limited to, vitamin E, vitamin A palmitate, vitamin D, vitamin F, vitamin $B_6$, vitamin $B_3$, vitamin $B_{12}$, vitamin C, ascorbyl palmitate, vitamin E acetate, biotin, niacin, DL-panthenol, and any combination of any of the foregoing.

Suitable amino acids include, but are not limited to, glycine, serine, and any combination of any of the foregoing.

Suitable adjuvants include, but are not limited to, aesthetic modifying agents. The composition of the current invention includes at least one or more aesthetic modifying agents. An aesthetic modifying agent is a material which imports desirable tactile, olfactory, taste or visual properties to the surface to which it is applied. These materials can either be hydrophobic or hydrophillic. The aesthetic modifier generally is a hydrophobe. Preferably the hydrophobe is oil, wax, solid or paste. The hydrophobic aesthetic modifiers which are used in the present invention are dispersed into an aqueous phase typically by ultra high shear mixing, microfluidization or any other method known in the art which can produce a commercially stable dispersion that is essentially free of emulsifying surface active agents. The dispersions of the present invention are produced by mixing from about 0.1% to about 70% hydrophobic aesthetic modifying agents or blends of aesthetic modifying agents with from about 30% to about 99.9% aqueous phase employing high pressure/ high shear conditions. This produces a homogenous fluid dispersion which is stable for a commercially relevant period of time. The preferred pressure for preparing the dispersion is between about 9,000 to about 25,000 psi with a desired shear that creates an average particle size of between about 50 to about 1000 nanometers.

## Exemplary-Embodiments of the Invention

### Example 1: Essential Fatty Acid ("EFA") Complex

A first composition was formed in two beakers. The wgt % values are calculated based on the wgt % of the resulting final composition, after the compositions of the beakers are combined.

In a first beaker was mixed 10 wgt % Emersol® 221 oleic acid, manufactured by Henkel Chemical Co.; 15 wgt % Emersol® 315 linoleic acid, manufactured by Henkel Chemical Co.; 5 wgt % industrene 120 liquid, linolenic acid enriched in coconut oil manufactured by CK Witco Chemical Co.; 0.05 wgt % ceramide III; 0.0001 wgt % phytospingosine, manufactured by Doosan Chemical Co.; and 0.05 wgt % cholesterol. The composition is heated to 80°C in a water bath and stirred until clear.

In a second beaker was mixed 54.3999 wgt % Dow Coming 345 fluid, a cyclomethicone; 10.00 wgt% Vitamin E; 5.00 wgt % alcolec BS, a lipid supplied by American Lecithin; and 0.50 wgt % liquapar PE, a mixture of phenoxyethanol, isopropylparaben and butylparaben, sold by Sutton. The composition was mixed until uniform at room temperature.

The first beaker was removed from heat and the contents of the first beaker were added to the second beaker. The second beaker was cooled to room temperature. The composition was then filtered through Whatman 1 paper.

The resulting composition was homogeneous.

### Example 2: Sansurf® EFA Composition

A composition was formed in two beakers. The wgt % values were calculated based on the basis of the wgt % of the resulting composition, after the two beakers were combined

In a first beaker, 60.95 wgt% distilled water; 25.00 wgt% of the EFA complex of Example 1; 10.0 weight% soybean oil; and 1.80 wgt% of Germazide MPB, were mixed.

In a second beaker, 0.25 wgt % Phosphlipon 90H (sold by American Lecithin Co.); and 2.00 wgt% basis LP 20H are mixed.

The contents of the second beaker were added to the first beaker, and the resulting composition was subjected to mixing with a Silverson high shear mixer until it was homogeneous, and was then processed through a M110 Microfluidiser, manufactured by Microfluidics, Inc. of Massachusetts, at from 9,000-25,000 psi.

### Example 3: SolarCat™ Composition

A first composition was formed in three beakers. The wgt % values were calculated based on the wgt % of the resulting final composition, after the compositions of the beakers are combined.

In a first beaker was mixed 25 wgt % Escalol 587, manufactured by Henkel Chemical Co.; 3.0 wgt % Escalol 567, manufactured by Henkel Chemical Co. The composition was heated to 75°C and stirred until clear.

In a second beaker was mixed 67.5 wgt % distilled water; 2.0 wgt % Germazide® MPB.

In a third beaker was mixed 2.5 wgt % Catemol S-180S, manufactured by Phoenix Chemical. The compositions in the second and third beaker were mixed at 75°C. The contents of the second beaker and third beaker were added to the first beaker, and the resulting composition was then processed through a M110 Microfluidizer, manufactured by Microfluidics, Inc. of Massachusetts, at from 9,000-25,000 psi.

## Example 4: Solarease® OS/B3 Composition

A first composition was formed in three beakers. The wgt % values are calculated based on the wgt % of the resulting final composition, after the compositions of the beakers are combined.

In a first beaker was mixed 25 wgt % Escalol 587, manufactured by Henkel Chemical Co.; 3 wgt % Benzophenone-3, manufactured by ISP Van Dyke. The composition was heated to approximately 80°C and stirred until clear.

In a second beaker was mixed 67.5 wgt % distilled water, 2.0 wgt % Germazide® MPB.

In a third beaker was mixed 2.5 wgt % Basis LP-20H, manufactured by Ikeda; 0.5 wgt % Phospholipon 80H manufactured by American Lecithin. The composition in the second beaker was subjected to mixing with a Silverson high shear mixer while slowly adding the composition of third beaker until it was homogeneous. The contents of the second beaker and third beaker were added to the first beaker, and the resulting composition was then processed through a M110 Microfluidizer, manufactured by Microfluidics, Inc. of Massachusetts, at from 9,000-25,000 psi.

## Example 5: Solarease® II Composition

A first composition was formed in five beakers. The wgt % values are calculated based on the wgt % of the resulting final composition, after the compositions of the beakers are combined.

In a first beaker was mixed 37.5 wgt % Escalol 557, manufactured by Henkel Chemical Co.; 10 wgt % Parsol 1789; 1.8 wgt % Silicone Based Lemon Balm Extract The composition was heated to approximately 75°C and stirred until dissolved The composition was then cooled to approximately 25°C.

In a second beaker was mixed 0.1 wgt % Disodium EDTA, manufactured by Spectrum; 0.4 wgt % Potassium Sorbate USP/NF, manufactured by Tri-K; 0.01 wgt % Phytic Acid manufactured by Sigma and 46.39 wgt % distilled water. The composition was then mixed in a separate vessel until all is dissolved.

In a third beaker was added 1.85 wgt % Germazide® MPB. The composition of the second and third beaker were mixed until homogenous.

In a fourth beaker was added 0.2 wgt % 99% TEA manufactured by Kramer Chemical. The pH of this composition was then adjusted to approximately 6.50.

In a fifth beaker was mixed 1.5 wgt % of Basis LP-20H manufactured by Ikeda and 0.25 wgt % Phospholipon 80H manufactured by American Lecithin.

The composition in the second beaker, third beaker and fourth beaker was subjected to mixing with a Silverson high shear mixer while slowly adding the composition of the fifth beaker until it was homogeneous. The contents of the first beaker was then added with the continuation of the mixing with a Silverson high shear mixer. The entire composition was then processed through a M110 Microfluidizer, manufactured by Microfluidics, Inc. of Massachusetts, at from 9,000-25,000 psi.

## Example 6: Sansurf® OMC Composition

A first composition was formed in one beaker. The wgt % values are calculated based on the wgt % of the resulting final composition, after the compositions of the beakers are combined.

In a beaker was mixed 45.90 wgt % distilled water; 28.57 wgt % Uvinul N-539-SG, manufactured by BASF; 21.53 wgt % Escalol 557 manufactured by Henkel Chemical Co.; 1.85 wgt % Germazide® MPB; 2.00 wgt % Basis LP-20H manufactured by Ikeda and .25 wgt % Phospholipon 80H manufactured by American Lecithin. The composition was subjected to mixing with a Silverson high shear mixer until it was homogeneous. The entire composition was then processed through a M110 Microfluidizer, manufactured by Microfluidics, Inc. of Massachusetts, at from 9,000-25,000 psi.

## Example 7: Sansurf® SPF-30

A first composition was formed in three beakers. The wgt % values are calculated based on the wgt % of the resulting final composition, after the compositions of the beakers are combined.

In a first beaker was mixed 25.0 wgt % Escalol 557, manufactured by Henkel Chemical Co.; 8.0 wgt % Escalol 567, manufactured by Henkel Chemical Co.; 6.0 wgt % Parsol 1789; and 12.5 wgt % Crodamol ISNP manufactured by Croda. The composition was heated to approximately 75°C and stirred until dissolved. The composition was then cooled to room temperature.

In a second beaker was mixed 44.0 wgt % distilled water; 2.0 wgt % Germazide® MPB; 0.25 wgt % Potassium Sorbate manufactured by Tri-K

In a third beaker was mixed 2.0 wgt % Basis LP-20H manufactured by Ikeda and 025 wgt % Phospholipon 80H manufactured by American Lecithin. The composition of the second and third beaker was subjected to mixing with a Silverson high shear mixer while slowly adding the composition of the first beaker until it was homogeneous. The entire composition was then processed through a M110 Microfluidizer, manufactured by Microfluidics, Inc. of Massachusetts, at from 9,000-25,000 psi.

The various commercially available products used in the exemplary embodiments and elsewhere in the application are described further below:

Germazide® MPB is a mixture of phenoxyethanol, chlorphenesin, glycerin, methylparaben, and benzoic acid and is available from Collaborative Laboratories, Inc. of East Setauket, NY.

Solarease® is a mixture of octylmethoxycinnamate, butyl methoxydibenzoylmethane, cyclomethicone, phospholipids, and water and is available from Collaborative Laboratories, Inc. of East Setauket, NY.

Seamollient® is a mixture of water, algae extract, chlorphenesin, propylene glycol, sodium dehydroacetate, and phenoxyethanol and is available from Collaborative Laboratories, Inc. of East Setauket, NY.

Sansurf® Cyclomethicone is a mixture of water, cyclopentasiloxane and phospholipids and is available from Collaborative Laboratories, Inc. of East Setauket, NY.

Solarease II is a mixture of octylmethoxycinnamate, butyl methoxydibenzoylmethane, cyclomethicone, phospholipids, and water and is available from Collaborative Laboratories, Inc. of East Setauket, NY.

Vitamin A & E liposomes is a mixture of water, phospholipids, tocopheryl acetate, and retinyl palpitate and is available from Collaborative Laboratories, Inc. of East Setauket, NY.

Sansurf® DMG is a mixture of water, petrolatum, dimethicone, perfluoropolymethylisopropylether, stearamidopropyl dimethylamine, stearic acid, and tocopherol acetate, and is available from Collaborative Laboratories, Inc. of East Setauket, NY.

Solarcat™ is a mixture of water, octyl methoxycinnamate, butyl methoxydibenzoylmethane, cyclomethicone, stearamidopropyl dimethylamine, stearamidopropyl dimethylamine stearate, and balm mint extract and is available from Collaborative Laboratories, Inc. of East Setauket, NY.

Catezomes™ OMC is a mixture of octyl methoxycinnamate and stearamidopropyl dimethylamine stearate and is available from Collaborative Laboratories, Inc. of East Setauket, NY.

Parsol 1789 is a butyl methoxydibenzoylmethane sold by Givaudan-Roure Specialty Division.

### Example 8: In-Vitro Sun Protection Factor ("SPF") Protocol

**Background:**

The SPF 290 is composed of an ultraviolet source, monochromator and a detector. The source is a 125 W xenon lamp that emits ultraviolet (UVB) and near ultraviolet (UVA) radiation. This radiation then is filtered and attenuated to accurately simulate a solar irradiance spectrum. The radiation passes through the sample where a portion of it is adsorbed. The light not adsorbed enters the integrating sphere, where it is collected and then enters the monochromator. The monochromator separates the light into discreet wavelength bands, which are picked up by the detector.

The SPF 290 feeds this information into a data acquisition card, which plugs into a computer. The computer uses this feedback to calculate SPF values for the sample. SPF is calculated using a series of equations. First, the monochromatic protection factor (MPF) is calculated as the reciprocal of transmittance. MPF =1/T. Transmittance in turn is the voltage feedback from the sample scan divided by the feedback from the reference scan. T = S/R. The value for sun protection factor is given by the equation

$$SPF = \Sigma E^* B / \Sigma (E^* B / MPF)$$

where E is the spectral irradiance of sunlight and B is the erythemal effectiveness. The software can calculate the standard deviation and mean of a series of MPF and SPF calculations.

**Sample Preparation:**

Cut a 5 cm x 7.5 cm piece of Transpore tape (3M Inc.) and place it on a glass slide using double sided tape (rough surface facing up). The sunscreen-containing agent is distributed across the rough surface of the Transpore tape evenly at a density of 2 ug/cm2. The only way to apply the agent is to pipette it in 10ul evenly spaced drops on the substrate. Once the sunscreen has been applied to the substrate, the material is rubbed into the tape with a gloved finger. The rubbing action should mimic the rubbing action on human skin in-vivo. Start a timer upon completion of the product rub in. Allow the substrate to sit for 20 minutes (dry down time).

The following table shows a product which can be formulated to make an SPF product.

| Product | Percentage Used | In-Vitro SPF |
| --- | --- | --- |
| Sansurf OMC B-3 | 25 | 23.92 |
| Sansurf OMC B-3 1789 | 30 | 43.06 |
| Solarease II | 20 | 22.1 |

**Claims**

1.  A method of forming a dispersion of a nonpolar or slightly polar physiologically active agent in an aqueous composition and which is free of emulsifying surfactants, said method comprising the steps of

    a) mixing said nonpolar or slightly polar physiological active agent with a solvent or cosolvent;
    b) combining said mixture with water;
    c) subjecting said combination to high pressure and/or high shear mixing to form a stable dispersion with a particle size of from about 50 to about 1000 nm.

2.  The method of claim 1 wherein said physiologically active agent is selected from the group consisting of retinol, butyl methoxydibenzoylmethane, benzophenone-3 or ceramides.

3.  The method of claim 1 wherein said solvent or cosolvent is selected from the group consisting of soybean oil, octylmethoxycinnamate, octyl salicylate and cyclomethicone.

4.  The method of claim 1 wherein said physiologically active agent is retinol and said solvent or cosolvent is soybean oil.

5.  The method of claim 1 wherein said physiologically active agent is butyl methoxydibenzoylmethane and said solvent or cosolvent is octylmethoxycinnamate.

6.  The method of claim 1 wherein said physiologically active agent is benzophenone-3 and said solvent or cosolvent is octyl salicylate.

7.  The method of claim 1 wherein said physiologically active agent is ceramide and said solvent or cosolvent is cyclomethicone.

8.  The method of claim 1, wherein said physiologically active agent does not form a stable dispersion in said aqueous composition in the absence of said high pressure and/or high shear mixing.

9.  A method of forming a dispersion of a nonpolar or slightly polar physiologically active agent in an aqueous composition and which is free of emulsifying surfactants, said method consisting of the steps of

    a) mixing said nonpolar or slightly polar physiological active agent with a solvent or cosolvent;
    b) combining said mixture with water;
    c) subjecting said combination to high pressure and/or high shear mixing to form a stable dispersion with a particle size of from about 50 to about 1000 nm.

10. A method of forming a dispersion of a nonpolar or slightly polar physiologically active agent in an aqueous composition and which is free of emulsifying surfactants, said method comprising the steps of

    a) mixing said nonpolar or slightly polar physiological active agent with a solvent or cosolvent selected from the group consisting of soybean oil, octylmethoxycinnamate, octyl salicylate and cyclomethicone;
    b) combining said mixture with water;
    c) subjecting said combination to high pressure and/or high shear mixing to form a stable dispersion with a particle size of from about 50 to about 1000 nm.

**Patentansprüche**

1. Verfahren zur Herstellung einer Dispersion aus einem nicht polaren oder leicht polaren physiologisch aktiven Mittel in einer wässrigen Zusammensetzung, welche keine emulgierenden oberflächenaktiven Mittel aufweist, wobei das Verfahren die folgenden Schritte umfasst:

   a) Mischen des nicht polaren oder leicht polaren physiologisch aktiven Mittels mit einem Lösungsmittel oder Co-Lösungsmittel;
   b) Kombinieren der Mischung mit Wasser;
   c) Unterwerfen der Kombination einem hohen Druck und/oder hohen Scher-Mischen um eine stabile Dispersion mit einer Teilchengröße von zwischen ungefähr 50 bis ungefähr 1000 nm zu bilden.

2. Verfahren nach Anspruch 1, wobei das physiologisch aktive Mittel aus der Gruppe ausgewählt wird, bestehend aus Retinol, Butylmethoxydibenzoylmethan, Benzophenon-3 oder Ceramiden.

3. Verfahren nach Anspruch 1, wobei das Lösungsmittel oder Co-Lösungsmittel aus der Gruppe ausgewählt wird, bestehend aus Sojabohnenöl, Octylmethoxycinnamat, Octylsalicylat und Cylcomethicon.

4. Verfahren nach Anspruch 1, wobei das physiologisch aktive Mittel Retinol ist und das Lösungsmittel oder Co-Lösungsmittel Sojabohnenöl ist.

5. Verfahren nach Anspruch 1, wobei das physiologisch aktive Mittel Butylmethoxydibenzoylmethan und das Lösungsmittel oder Co-Lösungsmittel Octylmethoxycinnamat ist.

6. Verfahren nach Anspruch 1, wobei das physiologisch aktive Mittel Benzophenon-3 und das Lösungsmittel oder Co-Lösungsmittel Octylsalicylat ist.

7. Verfahren nach Anspruch 1, wobei das physiologisch aktive Mittel Keramid ist und das Lösungsmittel oder Co-Lösungsmittel Cyclomethicon ist.

8. Verfahren nach Anspruch 1, wobei das physiologisch aktive Mittel keine stabile Dispersion in der wässrigen Zusammensetzung bildet, in Abwesenheit des hohen Druck und/oder hohen Scher-Mischens.

9. Verfahren zur Herstellung einer Dispersion aus einem nicht polaren oder leicht polaren physiologisch aktiven Mittel in einer wässrigen Lösung, welche keine emulgierenden oberflächenaktiven Mittel aufweist, wobei das Verfahren die Schritte umfasst:

   a) Mischen des nicht polaren oder leicht polaren physiologisch aktiven Mittels mit einem Lösungsmittel oder Co-Lösungsmittel;
   b) Kombinieren der Lösung mit Wasser;
   c) Unterwerfen der Kombination einem hohen Druck und/oder hohen Scher-Mischen, um eine stabile Dispersion mit einer Teilchengröße von zwischen ungefähr 50 bis ungefähr 1000 nm zu bilden.

10. Verfahren zur Bildung einer Dispersion eines nicht polaren oder leicht polaren physiologisch aktiven Mittel in einer wässrigen Zusammensetzung, welche keine emulgierenden oberflächenaktiven Mittel aufweist, wobei das Verfahren die Schritte umfasst:

   a) Mischen des nicht polaren oder leicht polaren physiologisch aktiven Mittels mit einem Lösungsmittel oder einem Co-Lösungsmittel gewählt aus der Gruppe bestehend aus Sojabohnenöl, Octylmethoxycinnamat, Octylsalicylat und Cyclomethicon;
   b) Kombinieren der Mischung mit Wasser;
   c) Unterwerfen der Kombination einem hohen Druck und/oder hohen Scher-Mischen, um eine stabile Dispersion mit einer Teilchengröße von ungefähr 50 bis ungefähr 1000 nm zu bilden.

**Revendications**

1. Procédé de formation d'une dispersion d'un agent physiologiquement actif, non polaire ou légèrement polaire, dans une composition aqueuse et qui ne contient aucun surfactant émulsifiant, ledit procédé comprenant les étapes de

a) mélange dudit agent physiologiquement actif non polaire ou légèrement polaire avec un solvant ou un cosolvant ;
b) combinaison dudit mélange avec de l'eau ;
c) soumission de ladite combinaison à haute pression et/ou fort cisaillement, pour former une dispersion stable ayant une granulométrie d'environ 50 à environ 1 000 nm.

**2.** Procédé selon la revendication 1, dans lequel ledit agent physiologiquement actif est choisi dans le groupe constitué par le rétinol, le butylméthoxydibenzoylméthane, la benzophénone-3 ou les céramides.

**3.** Procédé selon la revendication 1, dans lequel ledit solvant ou cosolvant est choisi dans le groupe constitué par l'huile de soja, le méthoxycinnamate d'octyle, le salicylate d'octyle et la cyclométhicone.

**4.** Procédé selon la revendication 1, dans lequel ledit agent physiologiquement actif est le rétinol, et ledit solvant ou cosolvant est l'huile de soja.

**5.** Procédé selon la revendication 1, dans lequel ledit agent physiologiquement actif est le butylméthoxydibenzoylméthane, et ledit solvant ou cosolvant est le méthoxycinnamate d'octyle.

**6.** Procédé selon la revendication 1, dans lequel ledit agent physiologiquement actif est la benzophénone-3, et ledit solvant ou cosolvant est le salicylate d'octyle.

**7.** Procédé selon la revendication 1, dans lequel ledit agent physiologiquement actif est un céramide, et ledit solvant ou cosolvant est la cyclométhicone.

**8.** Procédé selon la revendication 1, dans lequel ledit agent physiologiquement actif ne forme pas de dispersion stable dans ladite composition aqueuse en l'absence dudit mélange sous haute pression et/ou en présence d'un fort cisaillement.

**9.** Procédé de formation d'une dispersion d'un agent physiologiquement actif, non polaire ou légèrement polaire, dans une composition aqueuse et qui ne contient aucun surfactant émulsifiant, ledit procédé comprenant les étapes de

a) mélange dudit agent physiologiquement actif non polaire ou légèrement polaire avec un solvant ou un cosolvant ;
b) combinaison dudit mélange avec de l'eau ;
c) soumission de ladite combinaison à haute pression et/ou fort cisaillement, pour former une dispersion stable ayant une granulométrie d'environ 50 à environ 1 000 nm.

**10.** Procédé de formation d'une dispersion d'un agent physiologiquement actif, non polaire ou légèrement polaire, dans une composition aqueuse et qui ne contient aucun surfactant émulsifiant, ledit procédé comprenant les étapes de

a) mélange dudit agent physiologiquement actif non polaire ou légèrement polaire avec un solvant ou un cosolvant choisi dans le groupe constitué par l'huile de soja, le méthoxycinnamate d'octyle, le salicylate d'octyle et la cyclométhicone ;
b) combinaison dudit mélange avec de l'eau ;
c) soumission de ladite combinaison à haute pression et/ou fort cisaillement, pour former une dispersion stable ayant une granulométrie d'environ 50 à environ 1 000 nm.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9814174 A

- US 6004566 A

**Non-patent literature cited in the description**

- **Maibach H I.** Surfactants and experimental irritant contact dermatitis. *Contact Dermatitis,* October 1995, vol. 33 (4), 217-25

- **Barany E ; Lindberg M ; Loden M.** Biophysical characterization of skin damage and recovery after exposure to different surfactants. *Contact Dermatitis,* February 1999, vol. 40 (2), 98-103